Europäisches Patentamt

European Patent Office

Office européen des brevets

(11) Veröffentlichungsnummer: **0 264 790**
**A2**

## (12) EUROPÄISCHE PATENTANMELDUNG

(21) Anmeldenummer: 87114968.8

(22) Anmeldetag: 13.10.87

(51) Int. Cl.4: **C12N 15/00** , C12N 1/16

Der (Die) Mikroorganismus (Mikroorganismen) ist (sind) bei der Deutsche Sammlung für Mikroorganismen unter der (den) Nummer(n) DSM-14013 hinterlegt worden.

(30) Priorität: 22.10.86 DE 3635867

(43) Veröffentlichungstag der Anmeldung:
27.04.88 Patentblatt 88/17

(84) Benannte Vertragsstaaten:
AT BE CH DE ES FR GB GR IT LI LU NL SE

(71) Anmelder: **Boehringer Ingelheim International G.m.b.H**

**D-6507 Ingelheim am Rhein(DE)**

(72) Erfinder: **Hauptmann, Rudolf, Dr.**
**Döllachstrasse 22**
**A-2483 Ebreichsdorf(AT)**
Erfinder: **Spevak, Walter, Dr.**
**Ernstbrunnerstrasse 2/1/4**
**A-2000 Stockerau(AT)**
Erfinder: **Swetly, Peter, Prof. Dr.**
**Hietzinger Hauptstrasse 40B**
**A-1130 Wien(AT)**

(54) **Neue Hefeexpressionsvektoren für IFN-omega, Verfahren zu ihrer Herstellung und deren Verwendung.**

(57) Die vorliegende Erfindung betrifft neue Hefeexpressionsvektoren für IFN-omega, Verfahren zu ihrer Herstellung und deren Verwendung zur Herstellung von IFN-omega, vorzugsweise von IFN-omegal.

EP 0 264 790 A2

## Neue Hefeexpressionsvektoren für IFN-omega, Verfahren zu ihrer Herstellung und deren Verwendung

In Nucleic Acids Res. 13, 4739-4749 (1985) und in der EP-A-0.170.204 wird eine neue Klasse von Typ I Interferonen beschrieben, die als omega-Interferone bezeichnet werden, die für sie kodierenden DNA-Sequenzen, Plasmide, die diese DNA-Sequenzen enthalten, und die die neuen Interferone produzierenden Organismen.

Bei der Herstellung von größeren Versuchsmengen der neuen omega-Interferone, insbesondere von IFN-omegal, mittels den in den oben erwähnten Publikationen explizit beschriebenen Expressionsplasmiden, z.B. mit pRHWII oder pRHWI2 jeweils transformiert in E.coli HBIOI, zeigte es sich jedoch, daß es wünschenswert wäre, die Expression der neuen omega-Interferone, insbesondere die von IFN-omegal, zu steigern.

Desweiteren ist bekannt, daß die Expression von Typ I-Interferonen sowohl in Prokaryoten als auch in Eukaryoten (siehe Nature 293, 717-722 (1981) und The EMBO Journal I , 603-608 (1982)) normalerweise keine bemerkenswerten Unterschiede aufweist, das heißt, diese ist jeweils ungefähr gleich groß.

Überraschenderweise wurde nun gefunden, daß die Expression von IFN-omega, vorzugsweise von IFN-omegal, in Hefe um mindestens 3 Größenordnungen im Vergleich zu der in Bakterien wie in E.coli gesteigert wird.

Gegenstand der vorliegenden Erfindung ist somit ein verbessertes Verfahren zur Herstellung von IFN-omega, welches dadurch gekennzeichnet ist, daß Hefe, transformiert mit einem Hefevektor, der das Strukturgen für ein IFN-omega, vorzugsweise für IFN-omegal, unter Kontrolle eines Hefepromotors enthält, kultiviert und anschließend das exprimierte IFN-omega nach üblichen Methoden isoliert wird.

Ein weiterer Gegenstand der vorliegenden Erfindung sind die hierfür erforderlichen neuen mit den entsprechenden neuen Hefeexpressionsvektoren transformierten Hefen, die neuen Hefeexpressionsvektoren und Verfahren zu ihrer Herstellung. Diese weiteren Gegenstände der vorliegenden Erfindung erhält man erfindungsgemäß

a) durch Isolierung eines geeigneten Promotor-Fragmentes mittels Restriktionsenzymverdauung eines entsprechenden Hefevektors,

b) durch Linearisierung des für die Aufnahme eines Inserts geeigneten Hefevektors, wobei das Insert aus einem entsprechenden Promotor-Fragment und aus einem IFN-omega-Gen aufgebaut ist,

c) durch Isolierung des Strukturgens für IFN-omega durch Restriktionsenzymverdauung eines entsprechenden Plasmids und durch anschließende Ligierung des so erhaltenen Strukturgens mit dem gemäß a) hergestellen Promotor-Fragment,

d) durch Ligieren des gemäß c) hergestellten Fragments mit dem gemäß b) linearisierten Hefevektor,

e) durch Transformation des gemäß d) hergestellten Hefevektors und durch anschließende Auswahl des gewünschten Klons und

f) durch Transformation der gemäß e) erhaltenen Plasmid-DNA in Hefe und durch Replikation der die Plasmid-DNA enthaltenden Klone.

Erfindungsgemäß kann jedes Plasmid, das einen Hefe-kompatiblen Promotor, einen Transkriptionsterminator sowie einem Hefereplikationsursprung enthält, verwendet werden. Beispielsweise kommt als geeignetes Plasmid das Plasmid YRp7 (siehe Stinchcomb et al. in Nature 282, 39 (1979); Kingsman et al. in Gene 7, 141 (1979); Tschumper et al. in Gene 10, 157 (1980)) oder das Plasmid YEp 13 (siehe Broach et al. in Gene 8, 121-133 (1979)), das das Hefe-Gen LEU 2 enthält, das zur Ergänzung einer LEU -2-minus-Mutante verwendet werden kann, in Betracht.

Desweiteren kommen als geeignete Promotor-Sequenzen die 5'-flankierende Region der Gene für 3-Phosphoglycerat-Kinase (siehe Hitzeman et al. in J. Biol. Chem. 255, 2073 (1980)) oder für andere glykolytische Enzyme (Kawaski und Fraenkel, BBRC 108, 1107-1112 (1982)) wie Enolase, Glycerinaldehyd-3-phosphat-dehydrogenase, Triosephosphatisomerase (TPI) (siehe Alber et al. in J. Molec. Appl. Genet. I, 419-434 (1982)), Phosphoglyceratmutase, Aldulase, Hexokinase-I, Hexokinase-2, Pyruvat-Decarboxylase, Phosphofructokinase, Glucose-6-Phosphat-Isomerase, Phosphoglucose-Isomerase und -Glucokinase, Promotor-Regionen der Gene für Alkohol-Dehydrogenase-2 (siehe Williamson et al. in Nature 283, 214-216 (1980)), Isocytochrom C, Saure-Phosphatase, abbauende Enzyme, die mit dem Stickstoff-Metabolismus gekoppelt sind, und Enzyme, die für die Verarbeitung von Maltose und Galaktose verantwortlich sind, in Frage. Weiters können Promotoren, die durch den Hefe Mating Typ Locus reguliert werden, beispielsweise Promotoren der Gene BARI, MFαI, STE2, STE3, STE5 bei temperaturregulierten Systemen durch die Verwendung von temperaturabhängigen SIR Mutationen eingesetzt werden (Rhine PH.D. in Thesis, University of Oregon, Eugene, Oregon (1979), Herskowitz and Oshima, The Molecular Biology of the Yeast Saccharomyces, part I, 181-209 (1981), Cold Spring Harbor Laboratory).

Desweiteren kommen zur Kultivierung alle gebräuchlichen Hefen in Betracht, als besonders geeignet hat sich jedoch Saccharomyces cerevisiae vom Stamm GM-3C2 ($\alpha$, leu 2-3, leu 2-112, trp 1-1, his 4-519, cyc 1-1, cyp 3-1) (siehe G. Faye et al. in Proc. Natl. Acad. Sci. 78, 2258-2262 (1981)) erwiesen.

Erfindungsgemäß ist jedoch die Verwendung der 5'-flankierenden Region des ADHI-Gens (siehe G. Ammerer in Methods of Enzymology 101, 192-201 (1983)) als Promotor besonders bevorzugt, wobei dieses Fragment zusätzlich den Transkriptionsterminator und den Replikationsursprung des Hefe 2$\mu$ Plasmids enthält. Beispielsweise erfolgt die Herstellung des Hefeexpressionsvektors für IFN-omega1 wie folgt (siehe Abbildung):

a) Zur Herstellung des Hefe-ADHI-Promotor-Fragments wird ein den ADHI-Promotor enthaltendes Plasmid, vorzugsweise das Plasmid pESI03, mit BamHI und XhoI verdaut und anschließend das ca. 1450 Basenpaare lange Promotor-Fragment nach üblichen Methoden isoliert, z. B. mit Agarosegel-Chromatographie, Elektroelution und anschließende Präzipitation. Das hierfür verwendete Plasmid pESI03 erhält man durch Einbringen des ca. 1450 bp langen ADHI-Promotor-Fragmentes (BamHI-XhoI) aus dem Plasmid AX$\alpha$II (siehe G. Ammerer: Methods Enzymology 101, 192-201 (1983), Academic Press) in pUC 18 (siehe Pharmacia P-L, # 27-4949-01), welches durch die Einfügung eines XhoI Linkers in die HincII-Schnittstelle modifiziert wurde.

b) Ein geeigneter Hefevektor, z.B. ein geeignetes Plasmid, vorzugsweise das Plasmid pJDB207 (siehe V.D. Beggs: Gene cloning in yeast, Ed. R. Williamson: Genetic engineering Vol. 2, 175-203 (1981), Academic Press, London), welches in E.coli RRI unter der DSM-Nummer 3181 bei Deutsche Sammlung von Mikroorganismen, Grisebachstraße 8, D-3400 Göttingen, von der gleichen Anmelderin am 28. 12. 1984 hinterlegt wurde, wird für die Aufnahme eines Inserts, welches aus dem Hefe-ADHI-Promotor-Fragment und einem IFN-omega-Gen besteht, mit BamHI geschnitten und dadurch linearisiert. Anschließend werden zweckmäßigerweise die 5'-terminalen Phosphatgruppen entfernt, vorzugsweise mit Kalbsdarm-Phosphatase, um ein Religieren ohne Insert zu verhindern. Das so erhaltene linearisierte Plasmid wird nach üblichen Methoden isoliert, z. B. mittels Agarosegel-Chromatographie, Elektroelution und anschließender Präzipitation.

c) Zur Isolierung des Strukturgens für ein IFN-omega, vorzugsweise von IFN-omega1, wird ein für dieses Interferon kodierendes Plasmid, z. B. das Plasmid pRHW12 (siehe EP-A-0.170.204) mit HindIII linearisiert und anschließend die 5' überhängenden Enden in gerade Enden umgewandelt, vorzugsweise durch Zugabe des Klenow-Fragments der DNA-Polymerase. Die linearisierte Form des Plasmids wird nach Reinigung und Isolierung, vorzugsweise mit Agarosegelelektrophorese, mit XhoI-Linker versehen. Dies geschieht zweckmäßigerweise mit XhoI-Linkern (Pharmacia P-L) in Gegenwart von T4-Polynucleotidkinase und rATP. Nach Freisetzen der XhoI spezifischen 5'-Überlänge wird das linearisierte pRHW12 gereinigt und isoliert, z. B. mittels Agarosegelelektrophorase und Elektroelution.

Anschließend wird das Fragment, vorzugsweise vor dessen Präzipitation, mit dem gemäß a) hergestellten Fragment versetzt und nach Lösen der vereinigten Fragmente in einem geeigneten Puffer, z. B. TE-Puffer, ligiert, vorzugsweise mit T4-DNA-Ligase. Das so erhaltene ligierte Fragment wird anschließend mit BamHI geschnitten und nach üblichen Methoden gereinigt, z. B. mittels Agarosegelreinigung.

d) Das gemäß c) erhaltene BamHI-Fragment, welches den ADHI-Promotor und das IFN-omega-Gen enthält, wird mit einem geeigneten linearisierten Vektor, vorzugsweise mit dem gemäß a) hergestellten Vektor ligiert, vorzugsweise in Gegenwart von T4-DNA-Ligase.

e) E.coli HB101 wird mit der so hergestellten Ligaselösung, welche den gewünschten Vektor enthält, transformiert. Von den Klonen wird derjenige ausgewählt, welcher die gewünschte Konstruktion aufweist. Dieser wurde im Falle des IFN-omega-1-Strukturgens mit pY-JDB-HuIFN-omega1 bezeichnet.

f) Die gemäß e) erhaltene Plasmid-DNA wird in Hefe, vorzugsweise in Hefe vom Stamm GM-3C2, eingeschleust. Hierzu wird die cellulosehaltige Zellwand der Hefe enzymatisch entfernt. Die so erhaltenen nackten Zellen, die sogenannten Sphäroblasten werden dann einem Medium ausgesetzt, das neben der Plasmid-DNA noch Calciumchlorid und einen Alkohol wie Polyäthylenglykol enthält, der die Membran permeabel macht und so die Aufnahme der Plasmid-DNA ermöglicht. Anschließend werden die Sphäroblasten in Agar einbettet, damit sich ihre Zellwand regenerieren kann, und kultiviert.

Die gemäß f) erhaltene Hefekultur wird nach mindestens 40-stündiger Inkubation bei 30°C abzentrifugiert. Das während der Inkubation gebildete IFN-omega wird aus dem abzentrifugierten Pellet nach üblichen Methoden isoliert, z. B. durch Aufschluß mit Hilfe von Glaskugeln und anschließender Elution mit Guanidinium-hydrochlorid. Der CPE-Reduktionstest zeigte eine IFN-omega-Aktivität von mindestens $1 \times 10^8$ Einheiten/l Hefekultur.

Die überraschend hohe Synthese des IFN-omegal durch den neuen Hefeexpressionsklon konnte nicht vorhergesehen werden, da die Synthese des humanen IFN-omegal in E.coli etwa 3 Größenordnungen - schlechter erfolgt als die des IFN-α2(Arg) (siehe EP-A-0.II5.6I3). Dies ist umso überraschender, da die Konstruktion Promotor-ribosomale Bindungsstelle-Gen in den Klonen pRHWI2 (siehe EP-A-0.I70.204) und pER33 (siehe EP-A-0.II5.6I3) identisch ist. So ergibt auch die Einführung des IFN-α2(Arg)-Genes anstelle des IFN-omegal-Gens in das Plasmid pY-JDB-HuIFN-omegal eine Expression von I $\times$ I0$^8$ Einheiten IFN-α2-(Arg)/I.

Das nachfolgende Beispiel soll die Erfindung näher erläutern, ohne diese jedoch zu begrenzen:

Vorbemerkungen:

Das Konstruktionsschema für den IFN-omegal Hefeexpressionsvektor (siehe Abbildung) ist nicht maßstabgetreu.

Enzymreaktionen wurden laut Vorschrift der Hersteller durchgeführt, sonstige Techniken sind Standardtechniken und können z. B. dem Buch "Molecular cloning - a laboratory manual" von T. Maniatis (Cold Spring Harbor, I982) entnommen werden.

Bei der Hefetransformation wurden folgende Hefemedien verwendet:

YNB + CAA Medium (I Liter):
    6,7 g YNB ohne Aminosäuren
    5 g Casaminosäuren
    20 g Glucose
    Wasser

YPD Medium (I Liter):
    I0 g Hefeextrakt
    20 g Bacto-Peptone
    20 g Glucose
    Wasser
    20 ml 50 $^\times$ minus Leu drop out Lösung

50 $^\times$ minus Leu drop out Lösung (I00 ml):
    0,25 g Tryptophan
    0,20 g Arginin
    0,I0 g Histidin
    0,60 g Isoleucin
    0,40 g Lysin
    0,I0 g Methionin
    0,60 g Phenylalanin
    0,50 g Threonin
    0,I2 g Adenin
    0,24 g Uracil
    Wasser
    Sterilfiltrieren

Sorbit Platten (600 ml):
    I09,2 g Sorbit
    I2 g Glucose
    4,02 g YNB
    I5 g Agar
    Wasser
    Autoklavieren und auf 45°C abkühlen, dann die drop out Lösung zusetzen und ausplattieren

Topagar (I Liter):
    I82 g Sorbit
    20 g Glucose
    6,7 g YNB

25 g Agar
Wasser
Autoklavieren

SED (25 ml):
12,5 ml 2 M Sorbit
1,25 ml 0,5 M EDTA
11,25 ml dest. Wasser
192,75 mg DTT (Dithiothreit)
Sterilfiltration

SCE (25 ml):
12,5 ml 2 M Sorbit
5 ml 0,5 M Na$_3$citrat
0,5 ml 0,5 M EDTA
7 ml steriles, dest. Wasser

CAS (25 ml):
12,5 ml 2 M Sorbit
2,5 ml 100 mM CaCl$_2$
0,25 ml 1 M Tris pH 7,5
9,75 ml steriles, dest. Wasser

PEG Lösung (20 ml):
4 g PEG 4000 (Polyäthylenglykol, SERVA)
2 ml 100 mM CaCl$_2$
0,2 ml 1 M Tris pH 7,5
17,8 ml dest. Wasser
Sterilfiltrieren

SOG (5 ml):
2,5 ml 2 M Sorbit
1,7 ml YPD
0,3 ml 100 mM CaCl$_2$
12,5 $\mu$l 50 $\times$ minus Leu drop out Lösung
0,5 ml dest. Wasser
Sterilfiltrieren

Beispiel

Konstruktion eines Hefeexpressionsvektors für IFN-omega1

a) Herstellung des Hefe-ADHI-Promotor Fragmentes

80 $\mu$g des Plasmids pESI03 (hinterlegt in E.coli HB101 am 27. Februar 1987 bei der Deutschen Sammlung für Mikroorganismen unter der DMS-Nr. 4013 gemäß dem Budapester Vertrag) werden in 500 $\mu$l Lösung mit BamHI und XhoI doppelt verdaut. Das ca. 1450 Basenpaare (bp) lange Promotor Fragment wird über ein 1 % Agarosegel vom Vektor getrennt, aus dem Gel durch Elektroelution isoliert und präzipitiert. Das Fragment wird in 40 $\mu$l TE-Puffer (10 mM Tris, 1 mM EDTA, pH 8) aufgenommen.

## b) Vorbereitung des Vektors pJDB207

I0 µg pJDB207 werden in I00 µl Lösung mit BamHI geschnitten und dadurch linearisiert. Um ein Religieren in der Folge zu vermeiden, werden die 5'terminalen Phosphatgruppen durch Behandlung mit Kalbsdarm-Phosphatase (Calf intestine phosphotase, CIP) entfernt. Die lineare Form wird auf einem I % Agarosegel von eventuell nicht geschnittenem Plasmid getrennt, daraus durch Elektroelution isoliert und präzipitiert. Die Vektor-DNA wird in 20 µl TE-Puffer aufgelöst.

## c) Expressionsvektor für IFN-omegaI

50 µg Plasmid pRHWI2 werden in 600 µl Lösung mit HindIII linearisiert. Durch Zugabe des Klenow-Fragments der DNA-Polymerase I (I0 Einheiten) und den vier Desoxynukleosidtriphosphaten (je 25 µM) und Inkubation bei Raumtemperatur werden die 5' überhängenden Enden in gerade Enden umgewandelt. Die lineare Form des Plasmids wird durch Agarosegelelektrophorese und anschließender Isolierung gereinigt. Das Fragment wird in 50 µl TE-Puffer gelöst.

Um mit dem Promotor Fragment kompatible Enden zu erzeugen, müssen XhoI-Linker an die Enden des linearen pRHWI2 angebracht werden. 3 µl XhoI-Linker (0,06 $OD_{250\,nm}$, Pharmacia P-L, #27-7758-OI, Formel d[CCTCGAGG]) werden in 20 µl mittels 5 Einheiten T4-Polynukleotidkinase und rATP phosphoryliert. Nach Inaktivierung des Enzyms durch I0-minütiges Erhitzen bei 70°C werden 5 µl dieser Lösung mit I0 µl linearem pRHWI2 vereint und insgesamt 20 µl Reaktionslösung mit T4-DNA Ligase ligiert (I6 Stunden bei I4°C). Die Ligase wird durch I0-minütige Behandlung bei 70°C inaktiviert, und das Reaktionsvolumen mit I × Mediumpuffer (I0 mM Tris, pH 7,5, 50 mM NaCl, I0 mM $MgCl_2$) auf I50 µl gebracht.

Durch Behandlung mit I00 Einheiten XhoI werden die XhoI spezifischen 5' Überhänge freigesetzt. Das mit XhoI-Enden versehene, lineare pRHWI2 wird durch Agarosegelelektrophorese gereinigt und aus dem Gel elektroeluiert. Vor dem Präzipitieren werden 5 µl Promotorfragment (Punkt a) zugesetzt. Nach dem Präzipitieren werden die DNA Moleküle in I4,5 µl TE-Puffer aufgenommen und nach Zusatz des Ligasepuffers und 5 Einheiten T4-DNA-Ligase I6 Stunden bei I4°C ligiert. Nach Hitzeinaktivierung des Enzyms wird das Volumen der Lösung mit I × Mediumpuffer auf 200 µl gebracht, und die DNA mit BamHI geschnitten. Die gewünschte DNA wird durch Agarosegelreinigung erhalten und in 20 µl TE-Puffer gelöst.

## d) Ligieren der Fragmente

Der fertige Expressionsvektor entsteht durch Ligieren von 5 µl des BamHI-Fragmentes (Promotor und IFN-omegaI-Gen) mit I µl linearisiertem pJDB207 Vektor (Hefe 2µ Terminator und Hefe 2µ Replikationsursprung, Leu2 Marker, E.coli Replikationsursprung, Ampicillin Resistenzgen) in insgesamt I0 µl Lösung in Gegenwart von I unit T4-DNA Ligase.

## e) Transformation

I0 µl kompetente E.coli HBI0I Zellen werden durch Zusatz von 5 µl Ligaselösung transformiert und auf LB-Ager mit I00 µg/ml Ampicillin plattiert.

I2 der entstandenen Klone wurden willkürlich ausgesucht, und die darin enthaltenen Plasmide im Minimaßstab (I,5 ml Kulturen) isoliert. Nach Schnitt dieser Plasmide mit verschiedenen Restriktionsenzymen und Agarosegelelektrophorese wurde ein Plasmid ausgewählt, das die gewünschte Konstruktion aufweist, und mit pY-JDB-HuIFN-omegaI bezeichnet.

## f) Hefetransformation

Als Vorkultur werden 25 ml YPD mit einer Hefekolonie (Stamm GM3-C2) inokuliert. Die Kultur wird über Nacht bei 30°C wachsen gelassen. I00 ml YPD werden mit I00 µl der Vorkultur inokuliert und bei 30°C bis zu einer $OD_{600\,nm}$ von I wachsen gelassen (ca. $4 \times I0^7$ Zellen/ml). Die Hefezellen werden abzentrifugiert (5 Minuten bei 5000 rpm). Das Pellet wird in I0 ml SED resuspendiert und I0 Minuten bei 30°C inkubiert. Die Zellen werden nochmals abzentrifugiert (6 Minuten bei 2500 rpm). Die Zellen werden in I0 ml SCE resuspendiert.

Es werden 100 µl Glusulase (B-Glucuronidase, Arylsulfatase, Boehringer-Mannheim) zugesetzt und 30 Minuten bei 30°C inkubiert. Dabei entstehen die Sphäroblasten. Die Sphäroblasten werden abzentrifugiert (5 Minuten bei 2000 rpm). Das Pellet wird einmal mit CAS gewaschen und in 500 µl CAS resuspendiert.

120 µl Sphäroblasten werden mit 6 µg Plasmid-DNA (pY-JDB-HuIFN-omega1) versetzt und 15 Minuten bei Raumtemperatur inkubiert. Nach Zusatz von 1 ml PEG-Lösung wird weitere 15 Minuten bei Raumtemperatur inkubiert und anschließend die Zellen abzentrifugiert (5 Minuten bei 2000 rpm). Das Pellet wird in 150 µl SOG resuspendiert und 30 Minuten bei 30°C inkubiert. Danach werden 5 ml Topagar + 250 µl 50 $^\times$ minus Leu drop out Lösung zugesetzt, und die Zellen auf Sorbitplatten plattiert. Nach 2 bis 4 Tagen Inkubation bei 30°C entwickeln sich Kolonien.

## g) Hefe-Zellysate

25 ml YNB + CAA Medium werden mit einer transformierten Hefekolonie inokuliert. Die Inkubationsdauer beträgt zumindest 40 Stunden bei 30°C. 13 ml dieser Kultur werden 5 Minuten bei 4500 rpm abzentrifugiert. Das Pellet wird unter Vortexen in 0,5 ml 2 M Guanidinium-Hydrochlorid resuspendiert. Nach Zusatz von 2 ml Glaskugeln (Durchmesser: 0,4 bis 0,5 mm) wird 10 Minuten bei 4°C heftig geschüttelt. Danach wird der Überstand in ein Eppendorfgefäß umgefüllt und bei 0°C inkubiert. Die Glaskugeln werden einmal mit 0,5 ml 7 M Guanidinium-Hydrochlorid gewaschen, und die Waschlösung mit dem ersten Überstand vereint. Die Lösung wird 1 Minute in einer Eppendorf-Zentrifuge bei 12 000 rpm zentrifugiert. Der Überstand wird im CPE-Reduktionstest auf Interferonaktivität geprüft.

Ausbeute: 1 $^\times$ 10$^8$ Einheiten IFN-omega1/l Hefekultur

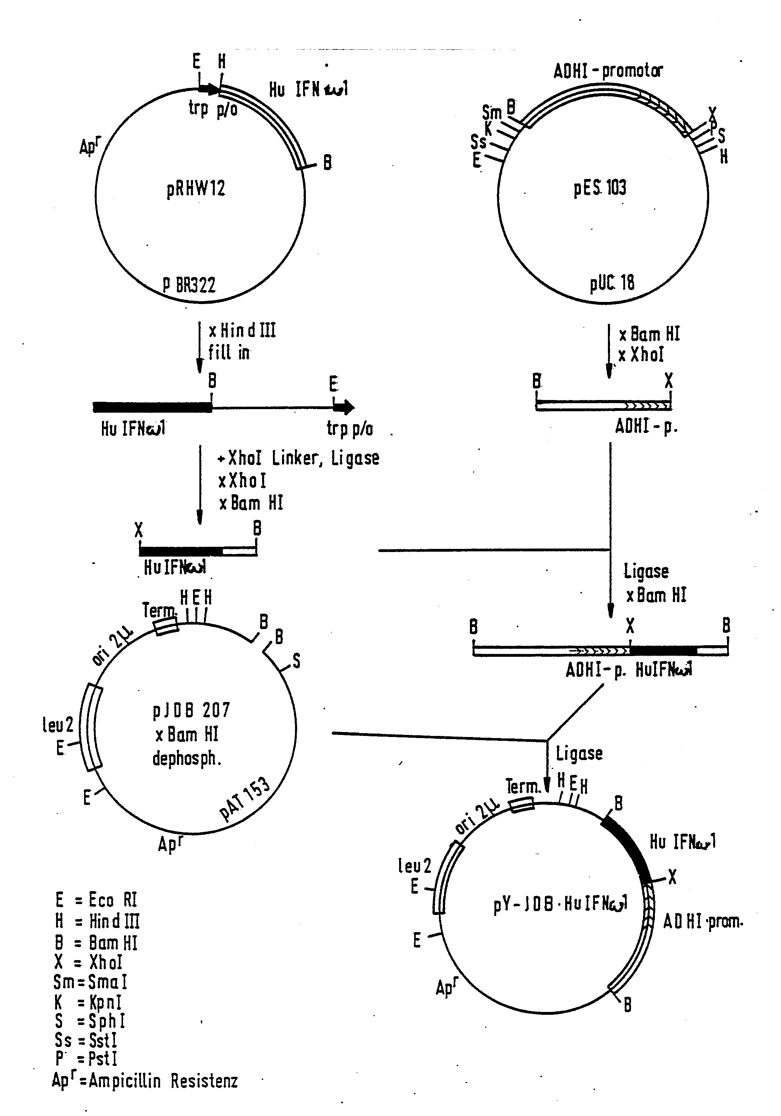

## Ansprüche

1. Verfahren zur Herstellung von IFN-omega, dadurch gekennzeichnet, daß mit einem Expressionsvektor transformierte Hefe, wobei der Expressionsvektor die genetische Information für ein IFN-omega und die für die Expression in Hefe erforderliche Replikations-und Kontrollsequenzen enthält, kultiviert wird, diese Information exprimiert und das so erhaltene IFN-omega isoliert wird.

2. Verfahren gemäß Anspruch 1, dadurch gekennzeichnet, daß der Expressionsvektor die genetische Information für IFN-omega1 enthält.

3. Verfahren gemäß Anspruch 1, dadurch gekennzeichnet, daß der Expressionsvektor den Promotor des ADHI-Gens, einen Transkriptionsterminator und Replikationsursprung des Hefe 2µ Plasmids enthält.

4. Verfahren gemäß den Ansprüchen 1 bis 3, dadurch gekennzeichnet, daß der Expressionsvektor die genetische Information für IFN-omega1 und den Hefe-ADHI-Promotor sowie einen Transkriptionsterminator und Replikationsursprung des Hefe 2µ Plasmids in der Weise enthält, daß die genetische Information exprimiert wird.

5. Verfahren gemäß Anspruch 4, dadurch gekennzeichnet, daß als Expressionsvektor das Plasmid pY-JDB-HuIFN-omega1 mit der Restriktionskarte

verwendet wird.

6. Hefeexpressionsvektor, enthaltend die genetische Information für ein IFN-omega und die für die Expression in Hefe erforderlichen Replikon-und Kontrollsequenzen.

7. Hefeexpressionsvektor gemäß Anspruch 6, dadurch gekennzeichnet, daß dieser die genetische Information für IFN-omegal enthält.

8. Hefeexpressionsvektor gemäß Anspruch 6, dadurch gekennzeichnet, daß dieser den Hefe-ADHI-Promotor sowie einen Transkriptionsterminator und Replikationsursprung des Hefe 2μ Plasmids enthält.

9. Hefeexpressionsvektor gemäß den Ansprüchen 6 bis 8, bezeichnet als pY-JDB-HuIFN-omegal mit der Restriktionskarte

10. Hefe transformiert mit einem Hefeexpressionsvektor gemäß den Ansprüchen 6, 7, 8, oder 9.

11. Hefe gemäß Anspruch 10, dadurch gekennzeichnet, daß Hefe vom Stamm GM3-C2 eingesetzt wird.

12. Verfahren zur Herstellung eines Hefeexpressionsvektors gemäß den Ansprüchen 6 bis 9, dadurch gekennzeichnet, daß die genetische Information für ein IFN-omega zusammen mit den erforderlichen Replikation-und Kontrollsequenzen in der Weise in einen Vektor eingefügt wird, daß diese Information exprimiert wird.

13. Verfahren zur Herstellung einer transformierten Hefe gemäß den Ansprüchen 10 oder 11, dadurch gekennzeichnet, daß Hefe mit einem Hefeexpressionsvektor gemäß den Ansprüchen 6, 7, 8 oder 9 transformiert wird.

E = Eco RI
H = Hind III
B = Bam HI
X = Xho I
Sm = Sma I
K = Kpn I
S = Sph I
Ss = Sst I
P = Pst I
Ap$^r$ = Ampicillin Resistenz